# EUROPÄISCHE PATENTSCHRIFT

(11) **EP 3 468 639 B1**
(45) Veröffentlichungstag und Bekanntmachung des Hinweises auf die Patenterteilung: **16.08.2023**
(21) Anmeldenummer: 17728199.5
(22) Anmeldetag: 07.06.2017
(51) Int. Cl.: A61M 5/142, A61M 5/168, A61M 5/20, A61J 1/20

(54) **VORRICHTUNG ZUR ABGABE EINES FLUIDS**
DEVICE FOR DISPENSING A FLUID
DISPOSITIF POUR LA DISTRIBUTION D'UN FLUIDE

(30) Priorität: 08.06.2016 EP 16173555
(43) Veröffentlichungstag der Anmeldung: 17.04.2019
(73) Patentinhaber: SHL Medical AG, 6300 Zug (CH)
(72) Erfinder: WEIBEL, Ludwig Daniel, 9104 Waldstatt (CH); WYLER, Samuel, 9030 Abtwil (CH)
(86) Internationale Anmeldenummer: PCT/EP2017/063749
(87) Internationale Veröffentlichungsnummer: WO 2017/211850

(56) Entgegenhaltungen:
- WO-A1-2014/090745
- US-A1- 2003 028 145

## Beschreibung

Die vorliegende Erfindung betrifft eine Vorrichtung zur Abgabe eines Fluids, insbesondere unter aseptischen Bedingungen, nach den Oberbegriffen des unabhängigen Anspruchs 1.

Bei der Verabreichung flüssiger Formulierungen pharmazeutischer Wirkstoffe ist es in den meisten Fällen erforderlich, wohl definierte Volumina an einen Patienten abzugeben. Häufig müssen Arzneimittel hierzu über eine Injektion verabreicht werden. Zur parenteralen Abgabe kommen dabei Injektionsspritzen, Arzneimittelstifte (sog. Pens) oder Arzneimittelpumpen zum Einsatz.

Insbesondere bei Präparaten, die über einen längeren Zeitraum und/oder nach einem genau vorgegebenen Schema verabreicht werden müssen, werden Spritzen und Pens immer mehr von Arzneimittelpumpen ersetzt. Ein Hauptanwendungsgebiet stellt dabei die Abgabe von Insulin an unter Diabetes leidende Patienten dar. Diabetiker müssen sich mehrmals täglich unter oft stark wechselnden Bedingungen selbst eine Insulindosis durch subkutane Injektion verabreichen. Der Einsatz einer Arzneimittelpumpe stellt eine deutliche Verbesserung ihrer Lebensqualität dar, da die Abgabe durch sie weitestgehend automatisiert werden kann. Neben einem verbesserten Komfort für den Patienten kann durch den Einsatz einer solchen Pumpe auch eine bessere Hygiene und damit eine höhere Patientensicherheit erzielt werden. Insulinpräparate sind hierzu besonders gut geeignet, da sie von den meisten Herstellern als fertige Injektionslösung in einer Karpulle bereitgestellt werden.

Arzneimittelpumpen, die dazu geeignet sind am Körper eines Patienten angebracht zu werden, um ein Präparat kontinuierlich über einen längeren Zeitraum zu verabreichen, umfassen in der Regel einen Behälter und eine Fördervorrichtung, welche das Fluid vom Behälter zu einem Anschluss oder zu einem Injektionssystem fördert. Allerdings können der Behälter und die Fördervorrichtung auch in einer Komponente vereint sein, beispielsweise in Form einer Spritzenpumpe. Ein Injektionssystem kann beispielsweise zum Setzen einer Verweilkanüle geeignet sein, die während des gesamten Verabreichungszeitraums im Körper eines Patienten verbleibt.

Derartige Arzneimittelpumpen sind in der Regel lediglich zur Verabreichung von fertigen Injektionslösungen geeignet. Sie weisen daher in Bezug auf die abzugebende Arzneimittelform eine vergleichsweise geringe Flexibilität auf.

Die WO 2014/090745 zeigt eine medizinische Pumpe zum Fördern einer Flüssigkeit. Die medizinische Pumpe umfasst eine Kammer, einen Einlass und einen Auslass. Die medizinische Pumpe umfasst ein erstes und ein zweites Kolbenelement, die beide beweglich in der Kammer angeordnet sind. Das erste und zweite Kolbenelement schliessen zwischen sich einen Raum ein, der innerhalb der Kammer durch Bewegen der Kolbenelemente verschiebbar ist.

Die europäische Patentanmeldung mit dem Aktenzeichen EP 15152703.3 offenbart ein Dosiergerät, welches eine von zumindest einem Förderantrieb angetriebene Fördervorrichtung zur Förderung eines Fluids aus dem Innenraum eines Behälters umfasst. Das Fluid ist mittels der Fördervorrichtung vom Behälter zu einer Abgabeöffnung förderbar. Die Fördervorrichtung umfasst einen Zylinder mit wenigstens einer Ansaugöffnung und wenigstens einer Auslauföffnung an einer Zylinderinnenwand, sowie einen ersten Kolben und einen zweiten Kolben. Der erste Kolben und der zweite Kolben sind innerhalb des Zylinders in Längsrichtung verschiebbar gelagert. Des Weiteren begrenzen der erste Kolben und der zweite Kolben zwischen ihren Stirnseiten gemeinsam mit einem Abschnitt der Zylinderinnenwand ein variables Fluidvolumen. Durch den Einsatz einer derartigen Fördervorrichtung mit einem Zylinder und zwei Kolben können mit dem beschriebenen Dosiergerät neben der reinen Förderfunktion weitere Funktionen wahrgenommen werden. So kann der Zylinder neben der Ansaugöffnung und der Auslassöffnung noch weitere Öffnungen aufweisen, die ebenfalls versetzt an ihm angeordnet sind. Damit ist es beispielsweise möglich, eine Analytikvorrichtung zur Analyse eines geförderten Fluids an die Fördervorrichtung anzuschliessen. Allerdings ist es auch möglich, mehrere Behälter über unterschiedliche Öffnungen mit der Fördervorrichtung zu verbinden, wodurch ein Mischen von Substanzen, beispielsweise zur Rekonstitution eines Lyophilisates, erzielbar ist.

Wie in der besagten Anmeldung beschrieben ist, umfassen die im Zylinder angeordneten Kolben jeweils zumindest drei in Längsrichtung versetzt angeordnete Dichtungen, um den Übergang von den Kolben zur Zylinderinnenwand dichtend abzuschliessen. Der Einsatz von Dichtungen hat den Vorteil,dass auch bei vergleichsweise geringen Fertigungstoleranzen von Zylinder und Kolben eine hohe Dichtwirkung erzielt werden kann. Es hat sich allerdings herausgestellt, dass ein derartiges Dosiergerät mit Dichtungen an den Kolben bei Anwendungen, bei welchen mehr als zwei versetzt am Zylinder angeordnete Öffnungen involviert sind, erhebliche Nachteile aufweist. So kann es beispielsweise vorkommen, dass zwei oder mehr Öffnungen am Zylinder über einen Zwischenraum zwischen zwei Dichtungen an einem Kolben direkt miteinander in Fluidkommunikation gebracht werden.Dadurch kann ein Überströmen eines Fluids von einer Öffnung zu einer benachbarten auftreten. Allerdings kann es auch bei Ausführungen des Dosiergerätes, bei denen ein Überströmen eines Fluids von einer Öffnung am Zylinder zu einer benachbarten aufgrund der Beabstandung der Dichtungselemente an den Kolben verhindert ist, zu einem indirekten Überströmen kommen. Dies kann dadurch erfolgen, dass Luft oder andere Gase, die sich in einem Zwischenraum zwischen zwei Dichtungen befinden, kompressibel sind, wodurch ein sich unter Druck befindendes Fluid in den Zwischenraum eindringen kann. Wird eine Verbindung zu einer Öffnung dann unterbrochen und eine zu einer benachbarten hergestellt, kann das komprimierte Substanzgemisch im Zwischenraum expandieren und aus der benachbarten Öffnung austreten. Diese Probleme stellen eine deutliche Beeinträchtigung der Funktionalität derartiger Dosiergeräte dar.

Es ist daher die Aufgabe der vorliegenden Erfindung, die Nachteile im Stand der Technik zu überwinden.

Insbesondere ist es eine Aufgabe der vorliegenden Erfindung, eine vielfältig anwendbare und konstruktiv einfache Vorrichtung zur Abgabe eines Fluids der oben genannten Art zu schaffen, bei der ein direktes oder indirektes Überströmen zwischen den Öffnungen am Zylinder der Fördervorrichtung verhindert ist. Dabei soll die Fördervorrichtung zuverlässig im Betrieb und zur Erzeugung vergleichsweise hoher Fluiddrücke geeignet sein.

Diese Aufgaben werden durch eine Vorrichtung gelöst, welche die Merkmale in Anspruch 1 aufweist.

Die Vorrichtung zur Abgabe eines Fluids, insbesondere unter aseptischen Bedingungen, umfasst eine Fördervorrichtung und einen Behälter mit einem variablen Innenvolumen. Die Fördervorrichtung umfasst einen Zylinder mit wenigstens drei der Reihe nach entlang einer Längsmittelachse angeordneten Öffnungen, über welche das Fluid förderbar ist. Darüber hinaus umfasst die Fördervorrichtung einen ersten und einen zweiten Kolben, wobei der erste und der zweite Kolben entlang der Längsmittelachse verschiebbar im Zylinder angeordnet sind. Der erste und der zweite Kolben begrenzen zwischen ihren Stirnseiten gemeinsam mit einem Abschnitt der Zylinderinnenwand ein variables Fluidvolumen. Die erste Öffnung ist mit einer Abgabeöffnung der Vorrichtung und die zweite Öffnung mit dem Behälter in Fluidkommunikation bringbar. Der erste und der zweite Kolben umfassen jeweils zumindest drei in Längsrichtung versetzt angeordnete Dichtungen, die den Zylinder dichtend abschliessen. Benachbarte Dichtungen an einem Kolben weisen dabei einen inneren Abstand auf, welcher höchstens gleich gross wie der kürzeste Abstand zwischen zwei benachbarten Öffnungen ist.

Unter einem Behälter mit einem variablen Innenvolumen wird in diesem Zusammenhang ein Behälter mit einem Innenraum verstanden, bei dem zumindest ein Wandabschnitt beweglich ist. Damit kann das Innenvolumen des Behälters dem Volumen des darin enthaltenen Fluids angepasst werden. Dies ist insbesondere vorteilhaft, wenn das Fluid in Form einer Flüssigkeit vorliegt. Einerseits kann so auf eine Belüftung des Behälters verzichtet werden. Andererseits enthält der Behälter, wenn er nicht vollständig mit einer Flüssigkeit gefüllt ist, kein Gas, insbesondere keine Luft. Dadurch wird ein Fördern der Flüssigkeit aus dem Behälter mittels der Fördervorrichtung unabhängig von der Orientierung der Vorrichtung gegenüber der Schwerkraft ermöglicht, ohne dass die Gefahr besteht, dass durch die Fördervorrichtung Luft oder anderes Gas angesaugt wird. Bei einem Behälter mit einem variablen Innenvolumen kann es sich zum Beispiel um eine Spritze oder Karpulle mit einem Schleppkolben oder um einen Flüssigkeitsbeutel (einen sogenannten Bag) handeln.

Durch die beschriebene Beabstandung von benachbarten Dichtungen an den Kolben in Relation zum kürzesten Abstand zwischen zwei benachbarten Öffnungen am Zylinder kann ein direktes Überströmen eines Fluids von einer Öffnung zur anderen über einen Zwischenraum zwischen den Dichtungen vermieden werden. Die beschriebene Vorrichtung ist dabei vielseitig für verschiedenste Anwendungen einsetzbar, welche über das reine Fördern eines Fluids hinausgehen, wie beispielsweise die zusätzliche Analyse eines Fluids mit einer Analytikvorrichtung oder die Rekonstitution eines Lyophilisates.

In einer einfachen Ausführungsform umfasst die Fördervorrichtung einen Zylinder mit genau drei der Reihe nach entlang seiner Längsmittelachse angeordnete Öffnungen sowie jeweils genau drei Dichtungen an den beiden Kolben.

Die Dichtungen an den Kolben können gleichmässig beabstandet sein. Dies ermöglicht es, eine gute Dichtwirkung und ein vorteilhaftes Gleitverhalten der Kolben innerhalb des Zylinders zu erzielen.

Der Abstand zwischen der ersten und der zweiten Öffnung kann mindestens gleich gross sein wie der seitliche Abstand zwischen zwei benachbarten Dichtungen der Kolben. Damit kann ein indirektes Überströmen eines Fluids von der zweiten Öffnung zur ersten vermieden werden. Dies ist insbesondere dann erforderlich, wenn ein Fluid zur zweiten Öffnung gefördert werden soll und sich dabei an der zweiten Öffnung ein positiver Druck aufbaut. Dies ist beispielsweise bei einem an die zweite Öffnung angeschlossenen elastischen Bag der Fall.

Die Dichtungen können eine Breite aufweisen, die zumindest gleich der Längsausdehnung, insbesondere der Durchmesser, der Öffnungen am Zylinder ist. Damit kann vermieden werden, dass ein Fluid über eine einzelne Öffnung von der einen Seite einer Dichtung zur anderen überströmen kann.

Der erste Kolben und der zweite Kolben können jeweils zumindest die gleiche Anzahl in Längsrichtung versetzt angeordnete Dichtungen umfassen, wie der Zylinder Öffnungen aufweist. Mit dieser minimalen Anzahl an Dichtungen kann gewährleistet werden, dass jeder einzelnen Öffnung am Zylinder stets ein zwischen zwei Dichtungen abgeschlossener Zwischenraum zur Verfügung steht, welches ein Ausströmen oder ein Überströmen eines Fluids verhindert.

Die Fördervorrichtung kann einen Zylinder mit vier der Reihe nach entlang seiner Längsmittelachse angeordneten Öffnungen umfassen, über welche das Fluid förderbar ist. Die dritte Öffnung kann mit einem Behälter, insbesondere einer Phiole, enthaltend ein Lyophilisat und die vierte Öffnung mit einem Behälter, insbesondere einer Phiole, enthaltend ein Lösungsmittel in Fluidkommunikation bringbar sein. Damit wird eine Vorrichtung geschaffen, welche insbesondere die Rekonstitution des Lyophilisates unter aseptischen Bedingungen auf eine für den Benutzer einfache und zuverlässige Weise ermöglicht.

Die Fördervorrichtung kann von zumindest einem Förderantrieb, vorzugsweise der erste Kolben von einem ersten Förderantrieb und der zweite Kolben von einem zweiten Förderantrieb, antreibbar sein. Insbesondere durch die Verwendung von zwei unabhängigen Förderantrieben wird die Flexibilität der Vorrichtung in Bezug auf ihre Anwendung weiter erhöht.

Die Vorrichtung kann eine Injektionsvorrichtung zur vorzugsweise kontinuierlichen subkutanen Abgabe eines Fluids an einen Patienten umfassen. Die Verwendung einer Injektionsvorrichtung ermöglicht es, die subkutane Abgabe des Fluids an einen Patienten weitestgehend zu automatisieren. Dadurch kann der Patient die besagte Vorrichtung unabhängig von der Anwesenheit von medizinischem Personal mit hohem Komfort und Sicherheit verwenden.

Die Vorrichtung kann ein Antriebsmodul und ein Abgabemodul umfassen, welche durch einen Benutzer verbindbar und/oder voneinander trennbar ausgebildet sind. Das Antriebsmodul kann gegebenenfalls zumindest Teile eines Förderantriebs, insbesondere einen Drehantrieb, und/oder gegebenenfalls einen Setzantrieb einer Injektionsvorrichtung umfassen. Darüber hinaus kann das Antriebsmodul auch eine Batterie zur Versorgung der Antriebe sowie eine Steuerungseinheit zur Steuerung der Vorrichtung, insbesondere der Antriebe umfassen. Ebenso können Kommunikationsmittel im Antriebsmodul vorgesehen sein, über welche eine externe Bedienungseinheit an die Steuereinheit anbindbar ist. Das Abgabemodul kann zumindest den Behälter sowie die Fördervorrichtung und gegebenenfalls die Injektionsvorrichtung umfassen.

Das Antriebsmodul und das Abgabemodul können derart ausgebildet sein, dass die Antriebe über entsprechende Kopplungsmittel auf einfache Weise direkt oder indirekt an die Fördervorrichtung und/oder die Injektionsvorrichtung ankoppelbar sind. Hierzu können zum Beispiel form- und/oder kraftschlüssige Steckkupplungen zum Einsatz kommen. Die beiden Module können zum einfachen Austausch beispielsweise über eine Schnappkupplung aneinander ankoppelbar und/oder trennbar sein.

Dies hat den Vorteil, dass das Antriebsmodul, welches keine hygienisch relevanten Komponenten enthält, vor seiner ersten Verwendung weder sterilisiert noch in einem Reinraum montiert werden muss. Damit können die Herstellungs- bzw. Anschaffungskosten der Vorrichtung reduziert werden. Zudem ist das Antriebsmodul problemlos wiederverwendbar, was ebenfalls die Betriebskosten der Vorrichtung senkt. Ausserdem bietet diese Ausgestaltung mehr Flexibilität bei konstruktiven Modifikationen. Das Abgabemodul, welches sämtliche hygienisch relevanten Komponenten enthält, muss zwar sterilisiert werden. Es kann jedoch, da es keine überaus komplexen Komponenten enthält, unter geringerem Kostenaufwand hergestellt und nach einmaligem Gebrauch entsorgt werden.

Da das Antriebsmodul und das Abgabemodul verbindbar und/oder voneinander trennbar ausgebildet sind, bleibt die Verwendung der Abgabevorrichtung durch einen Benutzer dennoch denkbar einfach. Als Resultat hiervon können bei gleichbleibender Bedienerfreundlichkeit und Patientensicherheit die Kosten von Anschaffung und Betrieb der Vorrichtung gesenkt werden.

Weitere Vorteile und Einzelmerkmale der Erfindung ergeben sich aus der nachstehenden Beschreibung eines Ausführungsbeispiels und aus den Zeichnungen.

Es zeigen schematisch:
- Figur 1:: Eine Fördervorrichtung aus dem Stand der Technik;
- Figuren 2 und 3:: Ein anderes Beispiel einer Fördervorrichtung aus dem Stand der Technik;
- Figur 4:: Perspektivische Darstellung einer erfindungsgemässen Vorrichtung;
- Figur 5a und 5b:: Vereinfachte Darstellung einer Fördervorrichtung einer erfindungsgemässen Vorrichtung;
- Figur 6:: Perspektivische Schnittansicht einer erfindungsgemässen Vorrichtung gemäss Figur 4;
- Figur 7:: Draufsicht auf die Fördervorrichtung gemäss den Figuren 4 und 6;
- Figur 8:: Seitenansicht der Fördervorrichtung gemäss den Figuren 4, 6 und 7;
- Figuren 9 bis 14:: Perspektivische Darstellung einer Sequenz von Schritten, welche die Rekonstitution eines Lyophilisates mit einer erfindungsgemässen Vorrichtung darstellt.

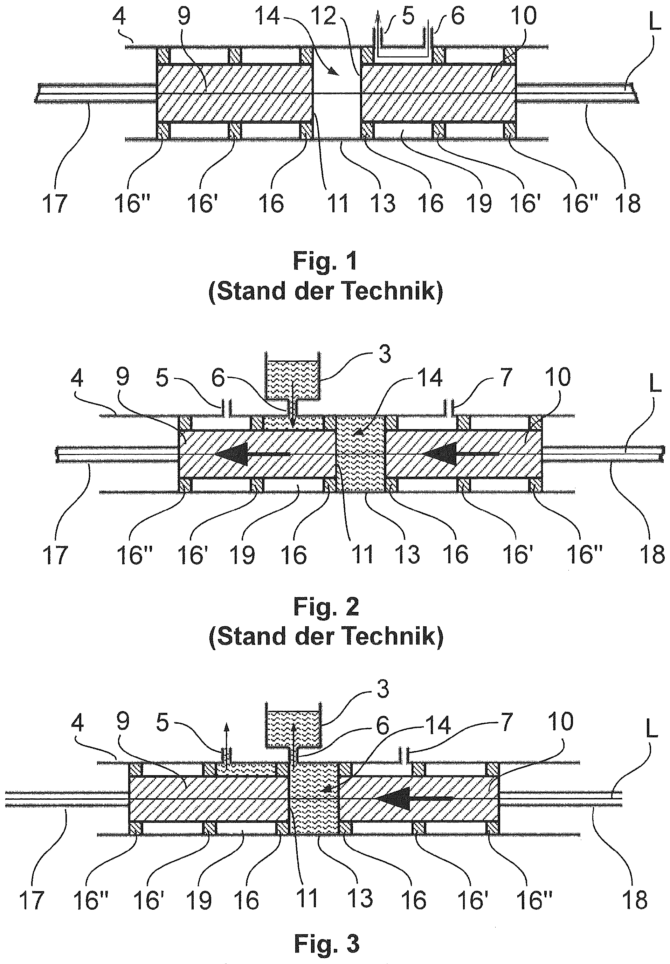

Figur 1 repräsentiert eine Fördervorrichtung aus dem Stand der Technik. Die besagte Fördervorrichtung umfasst einen Zylinder 4, in welchem die Kolben 9, 10 in Längsrichtung verschiebbar gelagert sind. Die Kolben 9, 10 schliessen mit ihren Stirnseiten 11, 12 sowie mit einer Zylinderinnenwand 13 ein variables Fluidvolumen 14 ein. Die Kolben 9, 10 weisen jeweils drei Dichtungen 16, 16', 16" auf, welche den Übergang zur Zylinderinnenwand 13 dichtend abschliessen. Die Kolben 9, 10 werden durch die Kolbenstangen 17, 18 angetrieben. Am Kolben 4 sind zwei entlang der Längsachse 11 versetzt angeordnete Öffnungen 5, 6 angebracht. Es ist zu erkennen, dass die Dichtungen 16, 16'an den Kolben 9, 10 derart beabstandet sind, dass je nach Position der zwischen den Dichtungen 16 und 16' abgeschlossene Zwischenraum 19 eine direkte Verbindung zwischen den Öffnungen 5 und 6 herstellt. Dadurch kann es, wie in der vorliegenden Darstellung gezeigt, zu einem unkontrollierten direkten Überströmen eines Fluids beispielsweise von der Öffnung 6 zur Öffnung 5 kommen.

Die Figuren 2 und 3 beziehen sich auf einen anderen nachteiligen Aspekt einer Fördervorrichtung aus dem Stand der Technik. Die besagte Vorrichtung ist derart ausgelegt, dass ein Fluid von der am Zylinder 4 angebrachten Öffnung 7 in einem ersten Schritt über die Öffnung 6 zum Behälter 3 förderbar ist. In einem zweiten Schritt ist ein Fördern des Fluids vom Behälter 3 zur Öffnung 5 vorgesehen. Wenn der Behälter 3 mit einem Fluid befüllt wird, baut sich an der Öffnung 6 ein Fluiddruck, beispielsweise ein hydrostatischer Druck, auf. In Figur 2 ist die besagte Vorrichtung beim gleichsinnigen, simultanen Verschieben der Kolben 9, 10 von der Öffnung 7 zur Öffnung 6 gezeigt. Es ist zu erkennen, dass bei diesem Vorgang die Öffnung 6 mit dem zwischen den Dichtungen 16 und 16' eingeschlossenen Zwischenraum 19 in Fluidkommunikation steht. Der Zwischenraum 19 ist mit einem Gas, üblicherweise mit Luft, gefüllt. Da das Gas kompressibel ist und an der Öffnung 6 ein Fluiddruck anliegt, kommt es zu einer Kompression des Gases und zu einem Eindringen des Fluids in den Zwischenraum 19. Die Figur 3 zeigt das Auslassen des Fluids vom Zwischenraum 14 in den Behälter 3. Der Kolben 9 wird dabei stationär gehalten, während der Kolben 10 auf diesen zubewegt wird, um das zwischen den Kolben 9, 10 eingeschlossene Volumen 14 zu verringern. Es ist zu erkennen, dass das zwischen den Dichtungen 16, 16' eingeschlossene Volumen 19 nun mit der Öffnung 5 in Fluidkommunikation steht. Da sich im Volumen 19 von dem in Figur 2 gezeigten Schritt noch ein komprimiertes Fluidgemisch befindet, kommt es zu einem unkontrollierten Austritt aus der Öffnung 5. Gesamthaft resultiert demnach ein unkontrolliertes indirektes Überströmen von Fluid von der Öffnung 6 zur Öffnung 5.

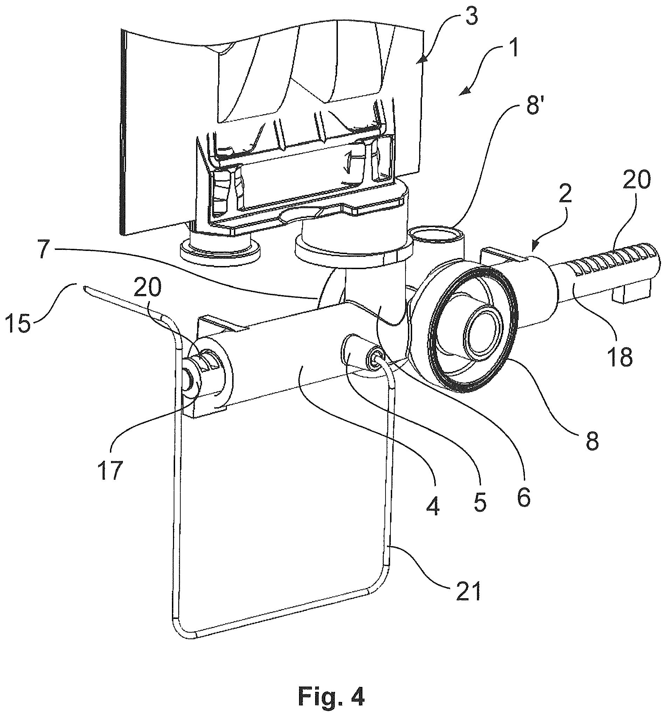

Die Figur 4 zeigt eine erfindungsgemässe Vorrichtung 1. Die besagte Vorrichtung 1 umfasst eine Fördervorrichtung 2 und einen Behälter 3, welcher hier als kollabierbarer Bag ausgebildet ist. Die Fördervorrichtung 2 setzt sich aus einem Zylinder 4 zusammen, in welchem die beiden Kolben 9, 10 in Längsrichtung verschiebbar gelagert sind. Die Kolben 9, 10 sind in der vorliegenden Darstellung nicht sichtbar. Jedoch sind die mit ihnen verbundenen Kolbenstangen 17 und 18 erkennbar. Im gezeigten Ausführungsbeispiel weist der Zylinder 4 insgesamt vier in Längsrichtung versetzt angeordnete Öffnungen 5, 6, 7, 8 auf. Die Öffnung 8` stellt hier eine Blindöffnung dar, bei der die Wand des Zylinders 4 nicht durchbrochen ist. Je nach Ausführung der Fördervorrichtung 2 kann ein Durchbruch bei der Öffnung 8 und/oder bei der Öffnung 8' vorliegen. Die Öffnung 5 ist über die Kanüle 21 mit der Abgabeöffnung 15 fluidmässig verbunden. Die Öffnung 6 steht mit dem Behälter 3 in Fluidkommunikation. Die Öffnungen 7 und 8 können ebenfalls mit Behältern, insbesondere mit Phiolen, in Fluidkommunikation gebracht werden.

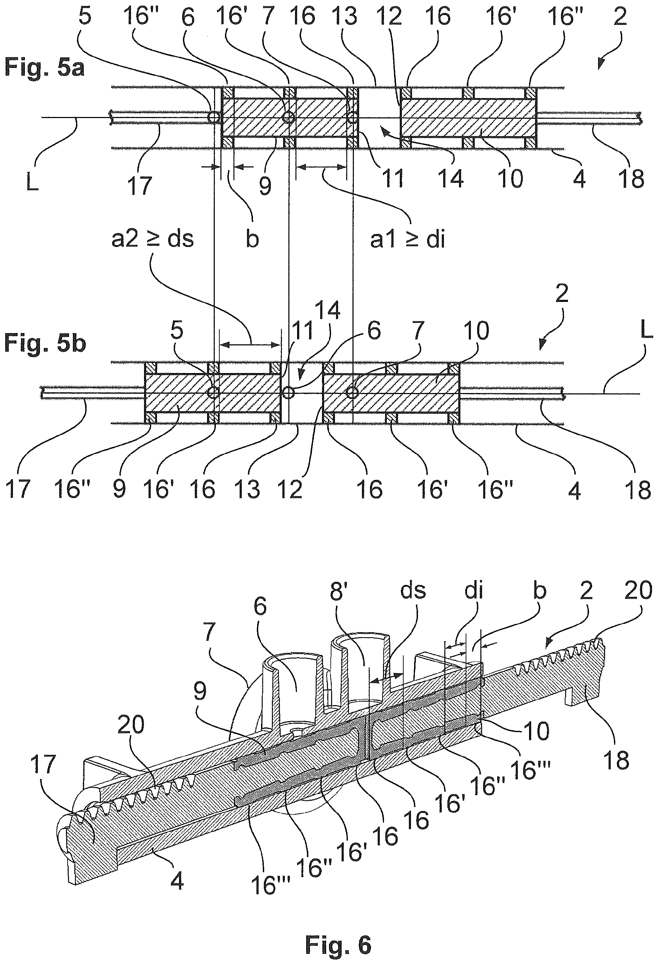

Die Figuren 5a und 5b zeigen eine vereinfachte Darstellung einer Fördervorrichtung 2 für eine erfindungsgemässe Vorrichtung 1. In der Figur 5a ist der innere Abstand di zwischen den Dichtungen 16 und 16' bzw. 16' und 16" derart gewählt, dass dieser höchstens gleich dem Abstand a1 zwischen der zweiten Öffnung 6 und der dritten Öffnung 7 ist. Eine direkte Fluidverbindung zwischen der Öffnung 6 und der Öffnung 7 kann damit nicht hergestellt werden, was ein unbeabsichtigtes Überströmen eines Fluids verhindert. Wie der Figur 5b zu entnehmen ist, ist der Abstand a2 zwischen der ersten Öffnung 5 und der zweiten Öffnung 6 derart gewählt, dass dieser grösser oder gleich dem seitlichen Abstand ds zwischen den Dichtungen 16 und 16' ist. Damit kann ein unbeabsichtigtes indirektes Überströmen eines Fluids von der Öffnung 6 zur Öffnung 5 vermieden werden, selbst wenn an der Öffnung 6 ein Fluiddruck anliegt. Die in den Figuren 5a und 5b gezeigte Fördervorrichtung 2 ist damit dazu geeignet, in einem ersten Schritt ein Fluid von der dritten Öffnung 7 zur zweiten Öffnung 6 zu fördern. In einem zweiten Schritt kann das Fluid von der zweiten Öffnung 6 zur ersten Öffnung 5 gefördert werden. Bei einer erfindungsgemässen Vorrichtung 1 ist die erste Öffnung 5 mit einer Abgabeöffnung der Vorrichtung 1 in Fluidkommunikation bringbar während die zweite Öffnung 6 mit einem Behälter in Fluidkommunikation bringbar ist.

Aus Figur 6 sind mehrere Einzelheiten einer Fördervorrichtung 2 gemäss der Figur 4 ersichtlich. Der Zylinder 4 umfasst insgesamt vier Öffnungen 5, 6, 7 und 8, von denen hier nur die Öffnungen 6 und 7 sichtbar sind. Die Öffnung 8` ist als Blindöffnung ausgeführt. Die beiden Kolben 9 und 10 bestehen aus einem elastischen Material und sind auf die Kolbenstangen 17 und 18 aufgesteckt. An jedem Kolben sind vier Dichtungen 16, 16', 16" und 16‴ einstückig ausgeführt. In der beschriebenen Darstellung sind sowohl der innere Abstand di zwischen zwei Dichtungen als auch der seitliche Abstand ds eingezeichnet. Zudem ist ersichtlich, dass eine einzelne Dichtung 16, 16', 16" und 16‴ eine breite b aufweist.

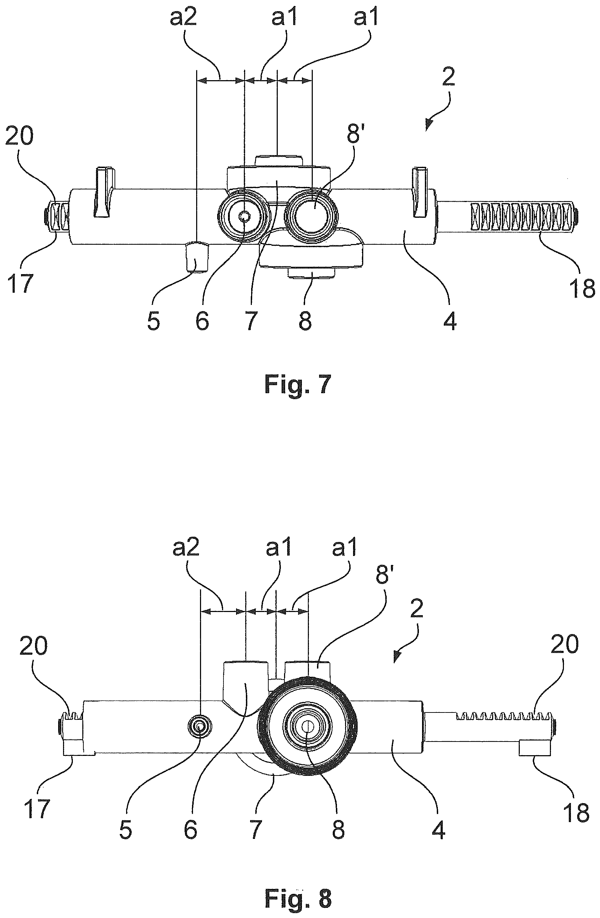

Die Figur 7 zeigt eine Draufsicht auf die Fördervorrichtung 2 einer erfindungsgemässen Vorrichtung 1 gemäss den Figuren 4 und 6. Aus dieser Perspektive sind alle vier Öffnungen 5, 6, 7 und 8 erkennbar. Zudem ist deutlich zu sehen, dass die Öffnung 8' als Blindöffnung ausgeführt ist. Der Abstand a1 zwischen der zweiten Öffnung 6 und der dritten Öffnung 7 ist gleich dem Abstand zwischen der dritten Öffnung 7 und der vierten Öffnung 8. Der Abstand a2 zwischen der ersten Öffnung 5 und der zweiten Öffnung 6 ist grösser als der Abstand a1. Die Figur 8 zeigt die Fördervorrichtung 2 gemäss der Figur 7 alternativ in einer Seitenansicht. Besonders gut sind hier die Zahnprofile 20 an den Kolbenstangen 17 und 18 zu erkennen, über welche ein hier nicht näher beschriebener Förderantrieb auf die Kolbenstangen 17 und 18 wirken kann.

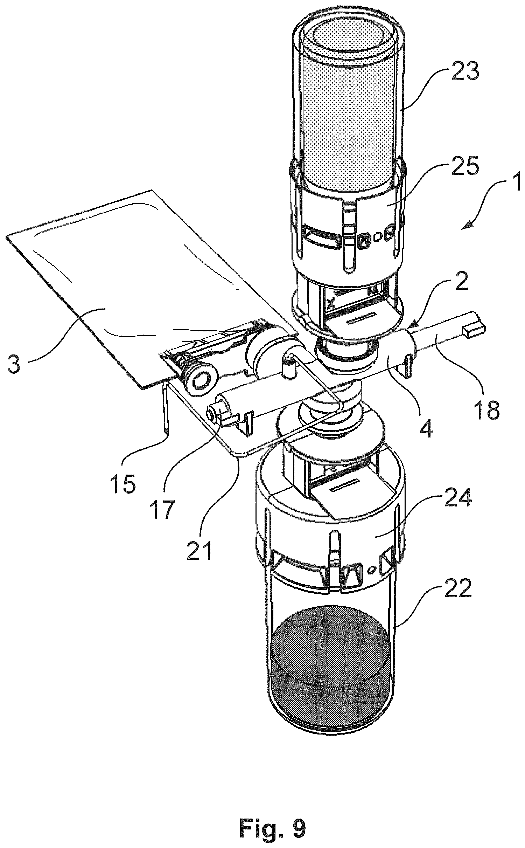

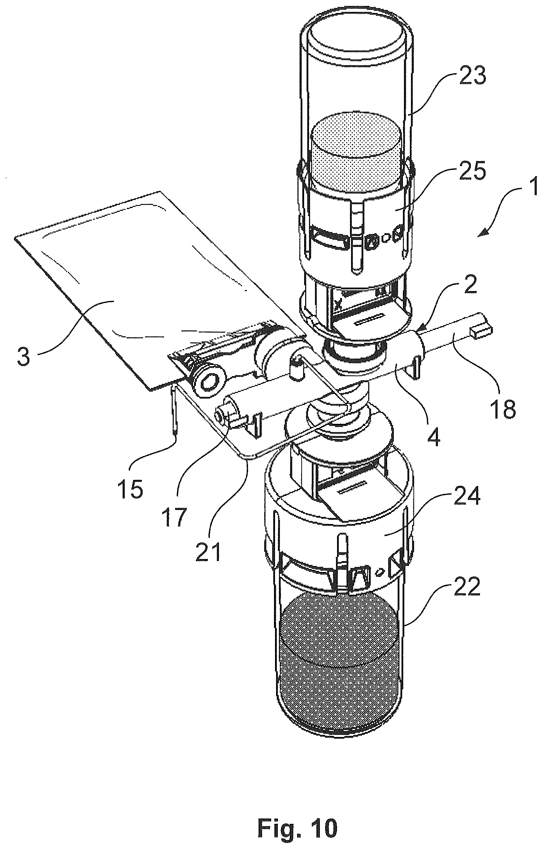

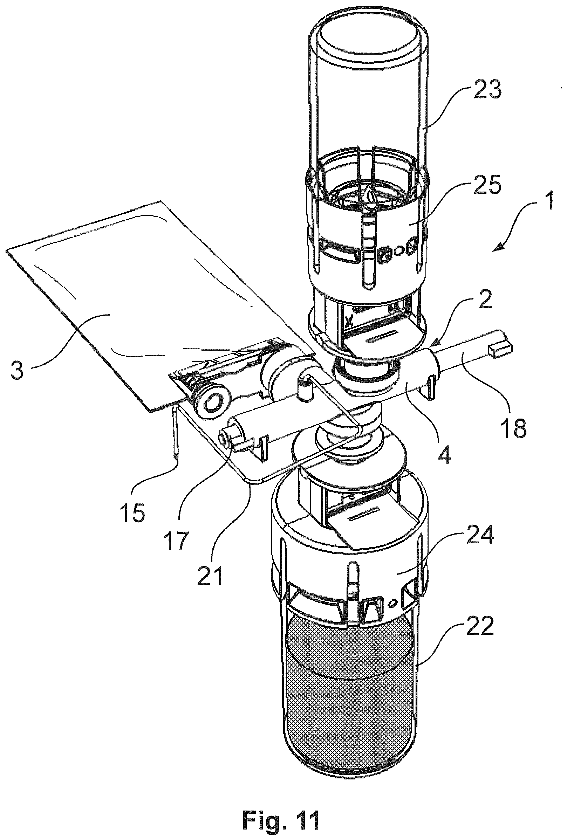

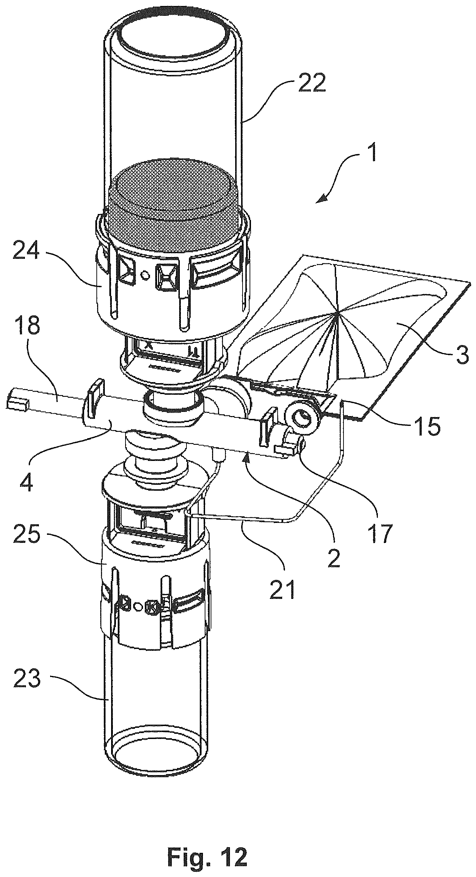

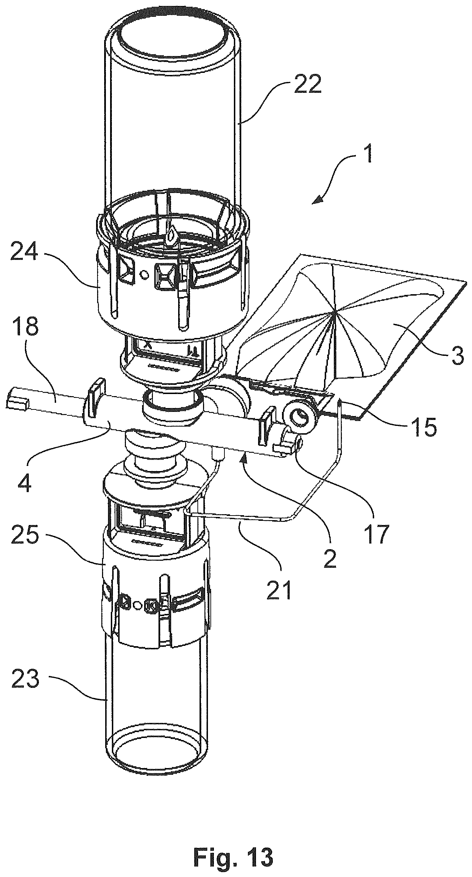

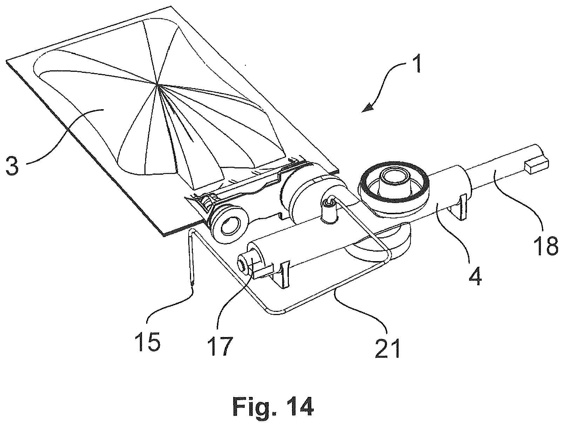

Die Figur 9 zeigt eine erfindungsgemässe Vorrichtung 1 gemäss den Figuren 4, 6, 7 und 8, an welche ein erster Behälter 22 mit einem Lyophilisat und ein zweiter Behälter 23 mit einem Lösungsmittel über Kopplungselemente 24 und 25 angeschlossen sind. In der Darstellung gemäss Figur 10 wird das im Behälter 24 vorhandene Lösungsmittel mit der Fördervorrichtung 2 von der Öffnung 8 zur Öffnung 7 gefördert, um das im Behälter 22 vorhandene Lyophilisat aufzulösen. In Figur 11 ist dieser Vorgang abgeschlossen. Sämtliches Lösungsmittel wurde vom Behälter 23 in den Behälter 22 gefördert, wo sich das Lyophilisat vollständig gelöst hat. In der Figur 12 wird die im Behälter 22 enthaltene Lösung durch die Fördervorrichtung 2 von der Öffnung 7 zur Öffnung 6 und damit zum Behälter 2 gefördert. Hierzu wird die Vorrichtung 1 um 180° gedreht, damit die im Behälter 22 vorhandene Lösung vollständig von der Fördervorrichtung 2 angesaugt werden kann. In Figur 13 ist der Transfer der Lösung vom Behälter 22 zum Behälter 3, welche hier als kollabierbarer Bag ausgebildet ist, abgeschlossen. Es ist zu erkennen, dass der Behälter 3 deutlich gebläht ist. Die Behälter 22 und 23 sowie die Kopplungselemente 24 und 25 können nun von der Vorrichtung 1 entfernt werden, wie es in Figur 14 gezeigt ist. Die Handhabung der Vorrichtung 1 zur eigentlichen Abgabe der Lösung, beispielsweise an einen Patienten, ist nun deutlich vereinfacht, da die Vorrichtung 1 weniger sperrig ist. Bei der eigentlichen Abgabe erfolgt ein Transfer der Lösung vom Behälter 3 über die Öffnung 6 zur Öffnung 5 und damit weiter über die Kanüle 21 zur Abgabeöffnung 15.

## Patentansprüche

1. Vorrichtung (1) zur Abgabe eines Fluids, insbesondere unter aseptischen Bedingungen, umfassend eine Fördervorrichtung (2) und einen Behälter (3) mit einem variablen Innenvolumen, wobei die Fördervorrichtung (2) einen Zylinder (4) mit wenigstens drei der Reihe nach entlang einer Längsmittelachse (L) angeordneten Öffnungen (5, 6, 7, 8) umfasst, über welche das Fluid förderbar ist, sowie einen ersten und einen zweiten Kolben (9, 10), wobei der erste und der zweite Kolben (9, 10) entlang der Längsmittelachse (L) verschiebbar im Zylinder (4) angeordnet sind und zwischen ihren Stirnseiten (11, 12) gemeinsam mit einem Abschnitt der Zylinderinnenwand (13) ein variables Fluidvolumen (14) begrenzen, und wobei die erste Öffnung (5) mit einer Abgabeöffnung (15) der Vorrichtung (1) und die zweite Öffnung (6) mit dem Behälter (3) in Fluidkommunikation bringbar sind, **dadurch gekennzeichnet, dass** der erste und der zweite Kolben (9,10) jeweils zumindest drei in Längsrichtung versetzt angeordnete Dichtungen (16, 16', 16") umfassen, die den Zylinder (4) dichtend abschliessen, wobei benachbarte Dichtungen(16, 16' bzw. 16', 16") an einem Kolben (9, 10) einen inneren Abstand (di) aufweisen, welcher höchstens gleich gross wie der kürzeste Abstand(a1) zwischen zwei benachbarten Öffnungen (5, 6 bzw. 6, 7) ist.

2. Vorrichtung (1) nach Anspruch 1, wobei die Dichtungen (16, 16', 16") an den Kolben (9, 10) gleichmässig beabstandet sind.

3. Vorrichtung (1) nach einem der Ansprüche 1 oder 2, wobei der Abstand (a2) zwischen der ersten und der zweiten Öffnung (5, 6) mindestens gleich gross wie der seitliche Abstand (ds) zwischen zwei benachbarten Dichtungen (16, 16' bzw. 16', 16") an den Kolben (9, 10) ist.

4. Vorrichtung (1) nach einem der Ansprüche 1 bis 3, wobei die Dichtungen (16, 16', 16") eine Breite (b) aufweisen, welche zumindest gleich der Längsausdehnung (d), insbesondere dem Durchmesser, der Öffnungen (5, 6, 7, 8) ist.

5. Vorrichtung (1) nach einem der Ansprüche 1 bis 4, wobei der erste und der zweite Kolben (9, 10) jeweils zumindest die gleiche Anzahl in Längsrichtung versetzt angeordnete Dichtungen (16, 16', 16") umfassen, wie der Zylinder (4) Öffnungen (5, 6, 7, 8) aufweist.

6. Vorrichtung (1) nach einem der Ansprüche 1 bis 5, wobei die Fördervorrichtung (2) einen Zylinder (4) mit vier der Reihe nach entlang seiner Längsmittelachse (L) angeordneten Öffnungen (5, 6, 7, 8) umfasst, über welche das Fluid förderbar ist, wobei die dritte Öffnung (7) mit einem Behälter (22), insbesondere eine Phiole, enthaltend ein Lyophilisat und die vierte Öffnung (8) mit einem Behälter (23), insbesondere einer Phiole, enthaltend ein Lösungsmittel in Fluidkommunikation bringbar ist.

7. Vorrichtung (1) nach einem der Ansprüche 1 bis 6, wobei die Fördervorrichtung (2) mit zumindest einem Förderantrieb, vorzugsweise der erste Kolben (9) von einem ersten Förderantrieb und der zweite Kolben (10) von einem zweiten Förderantrieb, antreibbar ist.

8. Vorrichtung (1) nach einem der Ansprüche 1 bis 7, umfassend eine Injektionsvorrichtung zur vorzugsweise kontinuierlichen subkutanen Angabe eines Fluids an einen Patienten.

9. Vorrichtung (1) nach einem der Ansprüche 1 bis 8, umfassend ein Antriebsmodul und ein Abgabemodul, welche durch einen Benutzer verbindbar und/oder voneinander trennbar ausgebildet sind, wobei das Antriebsmodul gegebenenfalls zumindest Teile des Förderantriebs, insbesondere einen Drehantrieb, und/oder gegebenenfalls einen Setzantrieb einer Injektionsvorrichtung umfasst, und das Abgabemodul zumindest den Behälter (3) sowie die Fördervorrichtung (2) und gegebenenfalls die Injektionsvorrichtung umfassen.

## Claims

1. Device (1) for dispensing a fluid, in particular under aseptic conditions, comprising a conveying device (2) and a container (3) having a variable internal volume, the conveying device (2) comprising a cylinder (4) having at least three openings (5, 6, 7, 8) arranged in a row along a longitudinal center axis (L), via which the fluid can be conveyed, and a first and a second piston (9, 10), the first and the second piston (9, 10) being arranged in the cylinder (4) so as to be displaceable along the longitudinal center axis (L) and delimiting a variable fluid volume (14) between their end faces (11, 12), together with a portion of the inner cylinder wall (13), and it being possible for the first opening (5) to be brought into fluidic communication with a dispensing opening (15) of the device (1) and for the second opening (6) to be brought into fluidic communication with the container (3), **characterized in that** the first and the second piston (9, 10) each comprise at least three seals (16, 16', 16") which are arranged offset in the longitudinal direction and close the cylinder (4) in a sealing manner, adjacent seals (16, 16' or 16', 16") on a piston (9, 10) having an inner distance (di) which is at most equal to the shortest distance (a1) between two adjacent openings (5, 6 or 6, 7).

2. Device (1) according to claim 1, wherein the seals (16, 16', 16") are uniformly spaced on the pistons (9, 10).

3. Device (1) according to either claim 1 or claim 2, wherein the distance (a2) between the first and the second opening (5, 6) is at least equal to the lateral distance (ds) between two adjacent seals (16, 16' or 16', 16") on the pistons (9, 10).

4. Device (1) according to any of claims 1 to 3, wherein the seals (16, 16', 16") have a width (b) which is at least equal to the longitudinal extension (d), in particular the diameter, of the openings (5, 6, 7, 8).

5. Device (1) according to any of claims 1 to 4, wherein the first and the second piston (9, 10) each comprise at least the same number of seals (16, 16', 16"), which are arranged offset in the longitudinal direction, as the cylinder (4) has openings (5, 6, 7, 8).

6. Device (1) according to any of claims 1 to 5, wherein the conveying device (2) comprises a cylinder (4) having four openings (5, 6, 7, 8) arranged in a row along the longitudinal center axis (L) thereof, via which the fluid can be conveyed, wherein the third opening (7) can be brought into fluidic communication with a container (22), in particular a vial, containing a lyophilizate, and the fourth opening (8) can be brought into fluidic communication with a container (23), in particular a vial, containing a solvent.

7. Device (1) according to any of claims 1 to 6, wherein the conveying device (2) can be driven by at least one conveying drive, preferably the first piston (9) can be driven by a first conveying drive and the second piston (10) can be driven by a second conveying drive.

8. Device (1) according to any of claims 1 to 7, comprising an injection device for preferably continuous subcutaneous dispensing of a fluid to a patient.

9. Device (1) according to any of claims 1 to 8, comprising a drive module and a dispensing module which are designed to be connectable and/or separable from one another by a user, wherein the drive module optionally comprises at least parts of the conveying drive, in particular a rotary drive, and/or optionally comprises a setting drive of an injection device, and the dispensing module comprises at least the container (3) and the conveying device (2) and optionally the injection device.

## Revendications

1. Dispositif (1) permettant la distribution d'un fluide, en particulier dans des conditions aseptisées, comprenant un dispositif d'acheminement (2) et un récipient (3) comportant un volume intérieur variable, dans lequel le dispositif d'acheminement (2) comprend un cylindre (4) comportant au moins trois orifices (5, 6, 7, 8) disposés en rangée les uns après les autres le long d'un axe médian longitudinal (L), par l'intermédiaire desquels le fluide peut être acheminé, ainsi qu'un premier et un second piston (9, 10), le premier et le second piston (9, 10) étant disposés de manière mobile le long de l'axe médian longitudinal (L) dans le cylindre (4) et délimitant entre leurs faces frontales (11, 12), conjointement avec une section de la paroi intérieure de cylindre (13), un volume de fluide variable (14), et le premier orifice (5) pouvant être amené en communication fluidique avec un orifice de distribution (15) du dispositif (1) et le deuxième orifice (6) pouvant être amené en communication fluidique avec le récipient (3), **caractérisé en ce que** le premier et le second piston (9, 10) comprennent respectivement au moins trois joints (16, 16', 16") disposés de manière décalée dans la direction longitudinale, lesquels ferment de manière étanche le cylindre (4), les joints adjacents (16, 16' ou 16', 16") présentant au niveau d'un piston (9, 10) un écartement intérieur (di) qui est au plus égal à l'écartement le plus court (a1) entre deux orifices adjacents (5, 6 ou 6, 7).

2. Dispositif (1) selon la revendication 1, dans lequel les joints (16, 16', 16") sont écartés de manière uniforme au niveau des pistons (9, 10).

3. Dispositif (1) selon l'une des revendications 1 ou 2, dans lequel l'écartement (a2) entre le premier et le deuxième orifice (5, 6) est au moins égal à l'écartement latéral (ds) entre deux joints adjacents (16, 16' ou 16', 16") au niveau des pistons (9, 10).

4. Dispositif (1) selon l'une des revendications 1 à 3, dans lequel les joints (16, 16', 16") présentent une largeur (b) au moins égale à l'extension longitudinale (d), en particulier au diamètre, des orifices (5, 6, 7, 8).

5. Dispositif (1) selon l'une des revendications 1 à 4, dans lequel le premier et le second piston (9, 10) comprennent respectivement au moins le même nombre de joints (16, 16', 16") disposés de manière décalée dans la direction longitudinale que le cylindre (4) ne présente d'orifices (5, 6, 7, 8).

6. Dispositif (1) selon l'une des revendications 1 à 5, dans lequel le dispositif d'acheminement (2) comprend un cylindre (4) comportant quatre orifices (5, 6, 7, 8) disposés en rangée les uns après les autres le long de son axe médian longitudinal (L), par l'intermédiaire desquels le fluide peut être acheminé, le troisième orifice (7) pouvant être amené en communication fluidique avec un récipient (22), en particulier une fiole, contenant un lyophilisat, et le quatrième orifice (8) pouvant être amené en communication fluidique avec un récipient (23), en particulier une fiole, contenant un solvant.

7. Dispositif (1) selon l'une des revendications 1 à 6, dans lequel le dispositif d'acheminement (2) peut être entraîné par au moins un entraînement d'acheminement, de préférence le premier piston (9) peut être entraîné par un premier entraînement d'acheminement et le second piston (10) peut être entraîné par un second entraînement d'acheminement.

8. Dispositif (1) selon l'une des revendications 1 à 7, comprenant un dispositif d'injection permettant l'administration sous-cutanée, de préférence continue, d'un fluide à un patient.

9. Dispositif (1) selon l'une des revendications 1 à 8, comprenant un module d'entraînement et un module de distribution qui sont conçus de manière à pouvoir être reliés l'un à l'autre et/ou séparés l'un de l'autre par un utilisateur, dans lequel le module d'entraînement comprend éventuellement au moins des parties de l'entraînement d'acheminement, en particulier un entraînement rotatif, et/ou éventuellement un entraînement de pose d'un dispositif d'injection, et le module de distribution comprend au moins le récipient (3) ainsi que le dispositif d'acheminement (2) et éventuellement le dispositif d'injection.
